# EUROPÄISCHE PATENTANMELDUNG

(11) **EP 3 733 226 A1**
(43) Veröffentlichungstag der Anmeldung: **04.11.2020**
(21) Anmeldenummer: 19020319.0
(22) Anmeldetag: 02.05.2019
(51) Int. Cl.: A61M 5/142, A61M 5/145

(54) **SPRITZENPUMPE**

(71) Anmelder: Veterinärmedizinische Universität Wien, 1210 Vienna (AT)
(72) Erfinder: Schramel, Johannes Peter, 8243 Pinggau (AT); Auer, Ulrike, 1220 Wien (AT)
(74) Vertreter: Peham, Alois

(57) **Zusammenfassung**

Die Erfindung betrifft eine Spritzenpumpe zur Betätigung einer Spritze, insbesondere einer Infusions- oder Injektionsspritze, wobei die Spritze einen zylindrischen Spritzenkörper mit Kolbenstopp und einen darin beweglichen Kolben samt Kolbenschaft umfasst, wobei in den Kolbenschaft eine Zahnstange eingefügt oder integriert ist, wobei die Spritzenpumpe eine Einspannvorrichtung zur Fixierung des Spritzenkörpers aufweist und wobei ein Motor vorgesehen ist, welcher über ein in die Zahnstange eingreifendes Zahnrad den Kolben antreibt.

## Beschreibung

Die Erfindung betrifft eine Spritzenpumpe.

Eine Spritze ist ein medizinisches Instrument, das insbesondere zur Verabreichung von Medikamenten, Flüssigkeit oder Nahrung, oder zur Entnahme von Körperflüssigkeiten oder - gewebe eingesetzt wird.

Kolbenspritzen bestehen aus einem zylindrischen Hohlraum, einem darin beweglichen Kolben und einer konus- oder zylinderförmigen Düse. An diese kann eine Hohlnadel oder ein Schlauch angeschlossen werden. Handelsübliche Größen reichen von 0,5 bis 100 ml Volumen.

Nach der Zahl der Anwendungsfälle werden Einwegspritzen und Mehrwegspritzen unterschieden.

Einwegspritzen sind in der Regel einzeln und steril verpackt. Sie bestehen aus Kunststoff, wie etwa Polypropylen oder Cycloolefin-Copolymeren. Bei Fertigspritzen ist die zu verabreichende Arzneimittellösung schon enthalten; einige Fabrikate sind mit einem Mechanismus ausgestattet, der die integrierte Kanüle nach der Injektion sicher einschließt.

Meist werden Spritzen manuell betätigt, d.h. das medizinische Personal presst im Anwendungsfall, nach dem Ansetzen der Spritze den Kolben mit dem Daumen in den Zylinder.

Aus dem Stand der Technik sind aber auch sogenannte Spritzenpumpen bekannt, Geräte, die den Kolben einer in das Gerät eingespannten Infusions- oder Injektionsspritze so vorwärts bewegen, dass eine zu applizierende Medikamentendosis oder eine Injektionslösung in den Körper eines Patienten gepumpt, d.h. gefördert werden kann.

Mit einer Spritzenpumpe können Injektionen genau dosiert werden, was insbesondere bei Dauerbehandlungen von Bedeutung ist.

Aus der DE 10 2009 012 449 A1 ist ein Verfahren zum Betrieb einer Spritzenpumpe bekannt, bei welchem der Kolben einer Spritze kontinuierlich motorisch angetrieben wird und dabei der translatorischen Vortriebsbewegung des Kolbens eine rotatorische Bewegung überlagert wird. Hierfür ist eine Bewegungsschraube mit dem Kolben der Spritze verbunden, die mit einer Einspannvorrichtung vor dem Gehäuse der Spritzenpumpe eingespannt ist.

Aufgabe der Erfindung ist es, einen einfachen Adapter vorzuschlagen, der möglichst mit herkömmlichen Kolbenspritzen zur Anwendung gelangen kann, und dessen Einsatz eine Kolbenbewegung in beide Richtungen ermöglicht.

Erfindungsgemäß geschieht dies mit einem Verfahren gemäß Anspruch 1.

Vorteilhafte Ausgestaltungen ergeben sich aus den Unteransprüchen.

Die Allgemeinanästhesie bei großen Säugetieren wie beipielsweise Tiger, Bären, Elefanten, Kühen oder Pferden stellt auch heute noch ein hohes Risiko für den Patienten dar. Die perioperative Sterblichkeit liegt beispielsweise bei Pferden etwa bei 0.4 - 0.8%. Dieser, im Vergleich zur Humanmedizin, hohe Wert ist vor allem durch die kritische Aufwachphase bedingt.

Pferde sind Fluchttiere und haben lange und empfindliche Beine die einen hohen Schwerpunkt und damit einen unsicheren Stand bei getrübtem Bewusstsein bewirken. Die Pferde werden in eine gepolsterte Aufwachbox gebracht, welche die Verletzungsgefahr minimieren soll. Es wird auch versucht, mit Seilen am Schweif und Kopf eine gewisse Stabilisierung des Standes zu versuchen, um wiederholte Stürze zu vermeiden.

Ab einem bestimmten Wachheitsgrad ist eine Beeinflussung des weiteren Verlaufes nicht mehr möglich, da es zu gefährlich für das Personal ist, sich dem Pferd zu nähern, um es zu beruhigen oder gegebenenfalls eine medikamentelle Unterstützung zu geben. Ein Betreten der Aufwachbox ist zu diesem Zeitpunkt nicht mehr möglich und es muss er weitere Verlauf abgewartet werden. Manche Pferde reagieren mit Panik und verletzen sich erheblich. Es kommt zu Beinbrüchen, Verletzung der Wirbelsäule etc. sodass trotz gelungener Operation eine Euthanasie des Pferdes unumgänglich wird. Die Ursache des wenig vorhersehbaren Verhaltens besteht in einem individuell verschieden raschen Abbau der Anästhetika wodurch es zu Imbalanzen der Wirkungen kommen kann. Besonders sensible Pferde reagieren unvorhersehbar mit Panik bzw. wiederholtem Aufstehen (Fluchtreflex) und niederstürzen.

Durch den Einsatz der erfindungsgemäßen Spritzenpumpe mit Fernauslösung können den Pferden oder anderen Tieren ohne Risiko für das Bedienpersonal Medikamente injiziert werden damit die Aufwachphase beeinflusst und kontrolliert werden kann.

Die Erfindung wird anhand von Figuren näher erläutert.

Es zeigen beispielhaft:
Fig. 1 eine dreidimensionale Ansicht des Zusammenwirkens der Kernelemente einer erfindungsgemäßen Spritzenpumpe.
Fig. 2 eine Explosivdarstellung der Elemente einer erfindungsgemäßen Spritzenpumpe
Fig. 3 eine erfindungsgemäße Einspannvorrichtung

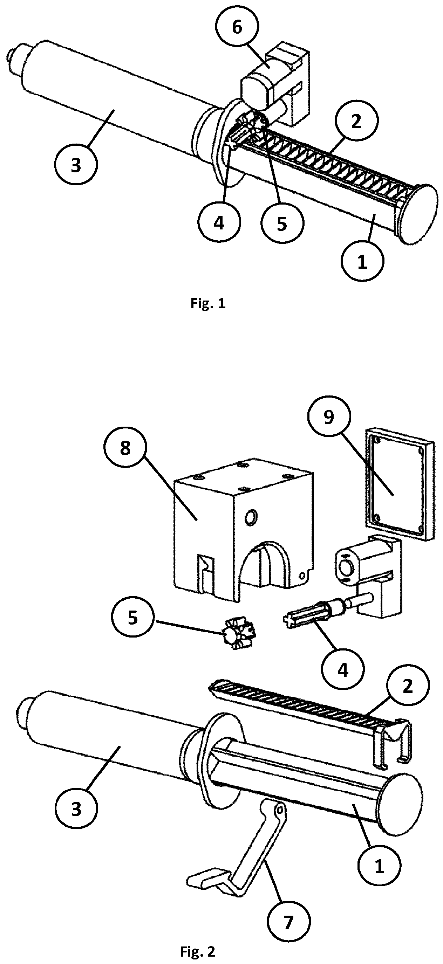

Wie in Fig. 1 dargestellt, umfasst die erfindungsgemäße Spritzenpumpe einen Motor 6, der über eine Zahnradachse 4 und ein Zahnrad 5 eine Zahnstange 2 antreibt, welche in einen Kolbenschaft 1 einer Spritze eingefügt ist.

Nicht dargestellt sind die elektronischen Elemente, welche eine Ansteuerung des Motors über Bluetooth Sende- und Empfangseinrichtungen gemäß dem IEEE 802.15.1 Standard ermöglichen. Dazu gehören insbesondere auch eine Batterie oder Akkumulator, sowie elektronische Steuerelemente zum Antrieb des Motors.

Dabei ist es auch denkbar, den Motor so anzusteuern, dass mit jedem Auslösevorgang der Kolben eine definierte Weglänge bewegt wird und damit eine vorbestimmte Flüssigkeitsmenge aus der Spritze gepresst und injiziert wird.

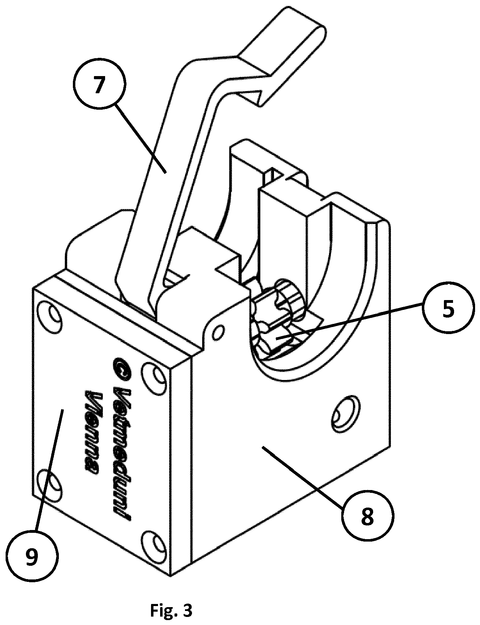

Wie in Figur 2 dargestellt, umfasst die erfindungsgemäße Spritzenpumpe neben den genannten Kernelementen auch eine in Figur 3 gesondert dargestellte Einspannvorrichtung 8, welche der Aufnahme des Motors 6 und der Fixierung des Spritzenkörpers 3 an der Spritzenpumpe dient. Die Einspannvorrichtung 8 umfasst weiterhin einen Gehäusedeckel 9 und eine Arretierklammer 7.

Zur Aufnahme des Spritzenkörpers weist die Einspannvorrichtung 8 eine Ausnehmung auf, in welche der Spritzenkörper 3 samt flügelförmigem Kolbenstopp einfügbar ist. Nach dem Einlegen des Spritzenkörpers 3 wird die Ausnehmung mit der Arretierklammer 7 formschlüssig verschlossen und die Spritze damit fixiert.

Alternativ können eine oder mehrere Zahnstangen in dem Kolbenschaft 1 bereits integriert sein.

Die erfindungsgemäße Spritzenpumpe kann besonders vorteilhaft bei Großsäugern, insbesondere bei Pferden eingesetzt werden.

Dazu können beispielsweise in eine gepolsterte Gesichtsmaske eine oder mehrere erfindungsgemäße Spritzenpumpen eingebaut werden, welche die vorbereiteten Injektabila auf ein ferngesteuertes Kommando abgeben können. Es können sowohl Sedativa als auch Schmerzmittel bei Bedarf verabreicht werden. Der Anästhesist bestimmt die Notwendigkeit, den Zeitpunkt, die Art und die Dosierung des Medikamentes. Dazu kann das Tier von Experten per Distanz oder über Video beobachtet werden. Optional kann mittels Elektroenzephalogramm die Narkosetiefe bzw. der Wachheitsgrad festgestellt werden. Weitere Informationen und Vitalparameter (Elektrokardiogramm, Atemfrequenz, Temperatur, Augenbewegungen, Pulsoximeter, Bewegungssensoren) können ebenfalls drahtlos an einen Monitor übertragen werden. Eine Sicherheitsvorrichtung sowie eine geschützte Datenübertragung verhindern eine unbeabsichtigte Verabreichung der Medikamente.

Alternativ können die Spritzenpumpen an anderen Körperregionen befestigt werden. Es ist darauf zu achten, dass die Vorrichtung keine Verletzungen verursacht und das Tier auch im Liegen und während der Aufstehphase nicht behindert wird.

In vorteilhaftere Weise können handelsübliche Einmalspritzen verwendet werden. Diese Spritzen weisen üblicherweise einen Kolbenschaft 1 mit kreuzförmigem Querschnitt auf, in den eine Zahnstange 2 eingefügt werden kann.

Die erfindungsgemäße Spritzenpumpe weist eine platzsparende Bauhöhe auf und ist damit einfach anzubringen.

Die Kontrolle der Abgabe der Flüssigkeit kann über ein Leuchtmittel erfolgen, dass bei Ansteuerung des Motors aktiviert wird.

Die Ansteuerung der Spritze erfolgt vorzugsweise über Bluetooth. Es sind aber auch andere Steuerungsverfahren wie beispielsweise Wi-Fi gemäß IEEE-802.11-Standard, optische Übertragungsverfahren etc. möglich.

### Bezugszeichenliste

- 1: Kolbenschaft
- 2: Zahnstange
- 3: zylindrischer Spritzenkörper
- 4: Zahnradachse
- 5: Zahnrad
- 6: Motor
- 7: Arretierklammer
- 8: Einspannvorrichtung
- 9: Gehäusedeckel

## Patentansprüche

1. Spritzenpumpe zur Betätigung einer Spritze, insbesondere einer Infusions- oder Injektionsspritze, wobei die Spritze einen zylindrischen Spritzenkörper mit Kolbenstopp und einen darin beweglichen Kolben samt Kolbenschaft umfasst, **dadurch gekennzeichnet, dass** in den Kolbenschaft (1) eine Zahnstange (2) eingefügt ist, dass die Spritzenpumpe eine Einspannvorrichtung (8) zur Fixierung des Spritzenkörpers (3) aufweist und dass ein Motor (6) vorgesehen ist, welcher über ein in die Zahnstange (2) eingreifendes Zahnrad (5) den Kolben antreibt.

2. Spritzenpumpe nach Anspruch 1, **dadurch gekennzeichnet, dass** in einem Kolbenschaft (1) eine oder mehrere Zahnstangen integriert sind.

3. Spritzenpumpe nach Anspruch 1, **dadurch gekennzeichnet, dass** Mittel zur drahtlosen Fernbedienung der Spritze vorgesehen sind.

4. Spritzenpumpe nach Anspruch 1, **dadurch gekennzeichnet, dass** die Einspannvorrichtung (8) zur Fixierung des Spritzenkörpers eine Ausnehmung aufweist, in welche der Spritzenkörper (3) samt Kolbenstopp einfügbar ist und eine Arretierklammer (7) zur Klemmung des Spritzenkörpers aufweist.

5. Spritzenpumpe nach Anspruch 1, **dadurch gekennzeichnet, dass** die Mittel zur drahtlosen Fernbedienung der Spritze Bluetooth Sende- und Empfangseinrichtungen gemäß dem IEEE 802.15.1 Standard umfassen.

6. Verfahren zum Einsatz einer Spritzenpumpe nach einem der Ansprüche 1 bis 4, **dadurch gekennzeichnet, dass** die Spritzenpumpe an einem Tier, insbesondere einem Pferd befestigt wird.
